Europäisches Patentamt

European Patent Office

Office européen des brevets

(11) Publication number : **0 570 079 A1**

# EUROPEAN PATENT APPLICATION

(21) Application number : 93201381.6

(22) Date of filing : 14.05.93

(51) Int. Cl.⁵ : **A01N 63/00,** C12N 1/20, A01C 1/06, // (A01N63/00, 25:08), (C12N1/20, C12R1:01)

The microorganism(s) has (have) been deposited with National Collection of Industrial Bacteria under number(s) NCIMB 40487 and 40488.

(30) Priority : 15.05.92 IT MI921172

(43) Date of publication of application : 18.11.93 Bulletin 93/46

(84) Designated Contracting States : AT BE CH DE DK ES FR GB GR IE IT LI LU MC NL PT SE

(71) Applicant : **MINISTERO DELL' UNIVERSITA' E DELLA RICERCA SCIENTIFICA E TECNOLOGICA**
**76, Lungotevere Thaon di Revel**
**I-00196 Roma (IT)**

(72) Inventor : **Noris, Emanuela**
**Corso G. Ferraris, 137**
**I-10128 Turin (IT)**
Inventor : **Signorini, Ernesto**
**Via Matteotti, 51**
**I-21046 Malnate, Varese (IT)**
Inventor : **Cassinelli, Clara**
**Via San Martino, 23**
**I-14040 Cortiglione, Asti (IT)**
Inventor : **Tolentino, Daniela**
**Via Marciano, 10**
**I-20133 Milan (IT)**

(74) Representative : **Roggero, Sergio**
**Ing. Barzanò & Zanardo Milano S.p.A. Via Borgonuovo 10**
**I-20121 Milano (IT)**

(54) **Azospirillum NCIMB 40487 and NCIMB 40488 active in biostimulation of plant growth.**

(57)    Disclosed are Azospirillum NCIMB 40487 and NCIMB 40488 and their use in the treatment of crops in order to increase corn plant growth.

EP 0 570 079 A1

The present invention relates to microorganisms of Azospirillum genus, identified as NCIMB 40487 and NCIMB 40488 and to their use in agricoltural field in order to stimulate corn plant growth.

The microorganisms of Azospirillum genus find their primary growth and diffusion substrate in soil in association with roots of Gramineae, Stonecrop, Composite, Papillionaceae, Nightshade and Cactus families.

We have found now two Azospirillum strains having particular morphological, biochemical and physiological characteristics, identified as NCIMB 40487 and NCIMB 40488 which, when added to the soil on which plants are grown, or used in order to dress seeds or anyway brought into contact with the roots of the plants, unexpectedly produce an improvement in vegetative growth and plant production.

Therefore, the subject-matter of the present invention is a method of treatment of corn crops in order to stimulate their growth, which method consists in treating the seeds, the roots or the soil with Azospirillum NCIMB 40487 and Azospirillum NCIMB 40488, as vegetative cells, as such or in the form of suitable compositions.

A subject-matter of the present invention also are Azospirillum NCIMB 40487 and NCIMB 40488, or their biologically pure cultures.

The morphological, biochemical and physiological characteristics of Azospirillum NCIMB 40487 and Azospirillum NCIMB 40488 made it possible them to be distinguished from Azospirillum strains known heretofore.

NCIMB 40487 strain, designated for internal laboratory use with the conventional code AZD 94, was isolated from a sample of an infestant root collected in an orchard at Badia Polesine, in the province of Rovigo. NCIMB 40488 strain, designated for internal laboratory use with the conventional code AZD 167, was isolated from a sample of an infestant root collected in an uncultivated land at Vidor in the province of Treviso.

The isolation of both strains was carried out on semisolid agarised MSP substrate having the following composition, as g/l:$K_2HPO_4$ 0.5; malic acid, 5; KOH, 4.5; $MgSO_4.7H_2O$, 0.2; $FeSO_4.7H_2O$, 0.5; NaCl, 0.1; $CaCl_2$, 0.02; $MnSO_4.H_2O$, 0.01; $Na_2MoO_4.2H_2O$, 0.002; bromothymol blue, 0.01; agar, 1.75. The MSP substrate is known to those skilled in the art, because it is selective to Azospirillum genus.

The cultures of such microorganisms where deposited on March 20th 1992, pursuant to the Treaty of Budapest, with the National Collection of Industrial Bacteria (c/o The National Collection of Industrial and Marine Bacteria Ltd., Torrey Research Station, P.O. Box 31, 135 Abbey Road, Aberdeen AB 98 DG, Scotland, - UK), where to them the accession numbers NCIMB 40487 and NCIMB 40488, respectively, were assigned.

The above identified microorganisms can be maintained on common substrates, such as, e.g., Nutrient Agar (OXOID Ltd.), or at -40°C in 10% dimethylsulfoxide or 20% glycerol and propagated by means of techniques per se known to those skilled in the art.

The growth temperature is generally comprised within the range of from 25°c to 37°C, with the temperature range comprised between 32°C and 37°C being anyway optimal.

Morphology and biochemical characteristics of Azospirillum NCIMB 40487 and NCIMB 40488

The taxonomic characterization of Azospirillum NCIMB 40487 and NCIMB 40488 strains was determined on the basis of morphological, physiological, biochemical characteristics and of characteristics of sensibility to antibiotics which made it possible them to be identified as Azospirillum brasilense, in accordance with the Bergey's Manual of Systematic Bacteriology, 1989.

In Table 1, the morphology of colonies of Azospirillum NCIMB 40487 and NCIMB 40488 strains after 5 days of incubation at 32°C on Nutrient Agar, is reported. The colonies generally have an irregular or rounded shape, a wrinkled appearance, of pink colour.

Table 1

| Characteristics | NCIMB 40487 | NCIMB 40488 |
|---|---|---|
| Size (mm) | 3-5 | 3-7 |
| Shape | circular | circular |

Table 1 (continuation)

| Characteristics | NCIMB 40487 | NCIMB 40488 |
|---|---|---|
| | -irregular | -irregular |
| Edge | indented | indented |
| Rellief | umbonate | umbonate |
| Surface | brilliant | brilliant |
| Transparency | not | not |
| | transparent | transparent |
| Consistency | creamy | creamy |
| Colour | pink | pink |

In Table 2 the morphology of the cells of Azospirillum NCIMB 40487 and NCIMB 40488 strains is reported after a 5-day incubation at 32°C on Nutrient Agar. The cells have a stab-like shape, are not sporogenic, are Gram-negative and very mobile in liquid culture media thanks to one single polar flagellum and have sizes of 1 micrometre of diameter x 2-4 micrometres of length.

Table 2

| Characteristics | NCIMB 40487 | NCIMB 40488 |
|---|---|---|
| Length (micrometres) | 2-4 | 2-4 |
| Width/diameter (micrometres) | 1 | 1 |
| Shape | stabs | stabs |
| Mobility | present | present |
| Arrangement | as single | as single |
| | cells | cells |
| Spore formation | absent | absent |

In the following Table 3, the physiological and biological essays on NCIMB 40487 and NCIMB 40488 strains are reported.

## Table 3

| Characteristics | NCIMB 40487 | NCIMB 40488 |
|---|---|---|
| Gram staining | − | − |
| Catalase | + | + |
| Oxidase | + | + |
| Gelatinase | − | − |
| Pectolytic capability (CVP substrate) | + | + |
| Urease | + | + |
| Growth on 5% NaCl | − | − |
| Indole production | − | − |
| Starch hydrolysis | − | − |
| Esculine hydrolysis | + | + |
| Growth on MacConkey's | − | − |
| Growth on Potato Dextrose Agar | + | + |
| $NO_3 - NO_2$ reduction | + | + |
| $NO_2 - N_2$ reduction | + | + |
| Use of: | | |
| Glycerol | + | ± |
| Erythrol | − | − |
| D-arabinose | − | − |
| L-arabinose | ± | ± |
| Ribose | − | − |
| D-xylose | − | − |
| L-xylose | − | − |
| Adonitol | − | − |
| Beta-methylxyloside | − | − |
| Galactose | − | − |

## Table 3 (continuation)

| Characteristics | NCIMB 40487 | NCIMB 40488 |
|---|---|---|
| Use of: | | |
| D-glucose | − | − |
| D-fructose | + | + |
| D-mannose | − | − |
| L-sorbose | − | − |
| Rhamnose | − | − |
| Dulcitol | − | − |
| Inositol | − | − |
| Mannitol | − | − |
| Sorbitol | ± | − |
| Alpha-methyl-D-mannoside | − | − |
| Alpha-methyl-D-glucoside | − | − |
| N-acetylglucosamine | − | − |
| Amygdalin | − | − |
| Arbutin | − | − |
| Salicin | − | − |
| Cellobiose | − | − |
| Maltose | − | − |
| Lactose | − | − |
| Melibiose | − | − |
| Saccharose | − | − |
| Trealose | − | − |
| Inulin | − | − |
| Melezitose | − | − |
| D-raffinose | − | − |
| Starch | − | − |
| Glycogen | − | − |
| Xylitol | − | − |

## Table_3 (continuation)

| Characteristics | NCIMB_40487 | NCIMB_40488 |
|---|---|---|
| Use_of: | | |
| Beta-gentiobiose | − | − |
| D-turanose | − | − |
| D-lixose | − | − |
| D-tagatose | − | − |
| D-fucose | − | − |
| L-fucose | − | − |
| D-arabitol | − | − |
| L-arabitol | − | − |
| Gluconate | − | − |
| 2-ketogluconate | ± | + |
| 5-ketogluconate | − | − |
| Anaerobic_acid_production | | |
| Glycerol | + | ± |
| L-arabinose | ± | ± |
| Xylose | ± | − |
| Fructose | + | + |
| Aerobic_acid_production | | |
| Glycerol | + | ± |
| L-arabinose | ± | ± |
| Fructose | − | + |
| Xylose | − | − |

In Table 4, the antibiogram of Azospirillum NCIMB 40487 and NCIMB 40488 strains is reported. The data were obtained by using the Sensi-Disks available from BBL (Becton Dickinson Microbiology Systems).

6

Table 4

| Antibiotic (BBL) | Dose (micrograms) | NCIMB 40487 | NCIMB 40488 |
|---|---:|---|---|
| Nalidixic acid | 30 | R(8)* | R(9) |
| Ampicillin | 10 | R | R |
| Bacitracin | 10 | R | R |
| Cephaloridine | 30 | R | R |
| Chloramphenicol | 30 | I(15) | I(16) |
| Chlorotetracycline | 30 | R(7) | R(5) |
| Erythromicyn | 15 | R(9) | R(12) |
| Fosfomycin | 50 | R | R |
| Gentamicin | 10 | R(8) | R(7) |
| Kanamycin | 30 | S(16) | S(15) |
| Lincomycin | 2 | R | R |
| Neomycin | 30 | R(8) | R(10) |
| Novobiocine | 30 | I(16) | I(15) |
| Oleandomicin | 15 | R | R |
| Oxytetracycline | 30 | R(14) | R(12) |
| Penicillin | 10 | R | R |
| Polymyxin B | 300 | R(3) | R(2) |
| Riphamycin | 30 | R(11) | R(11) |
| Rifampicin | 30 | S(17) | S(17) |
| Streptomycin | 10 | R(10) | I(14) |
| Tetracycline | 30 | R(5) | R(6) |
| Tobramycin | 10 | R(7) | R(8) |
| Vancomycin | 30 | R | R |

The values in brackets represent the diameter of inhibition halo. The evaluation of the sensibility degree, expressed by means of the definitions of resistant (R), intermediate (I) sensible (S), was carried out on the basis of the standard table recognized by NCCLS (National Committee for Clinical Laboratory Standards).

Cultivation characteristics

Azospirillum NCIMB 40487 and NCIMB 40488 strains can be cultivated in liquid substrate, keeping the cultures with agitation, or under stationary culture conditions on agarised substrate at a temperature preferably comprised within the range of from 25 to 35°C.

The initial isolates of Azospirillum NCIMB 40487 or NCIMB 40488 strains were obtained by suspending portions of plant roots previously washed in running water, in 0.01 M phosphate buffer and kept with stirring for 1 hour.

Aliquots of suspension were used in order to inoculate test tubes containing semisolid agarised substrate MSP, selective for Azospirillum genus, or CR substrate (MSP to which 15 g/l of agar and 0.1 g/l of Congo Red are added).

The test tubes were incubated at 32°C for 48 hours until an evident growth of the microorganism was obtained, with the formation of the typical skin of this genus and substrate alcalifying.

After 2-3 successive passages of skin portions inside test tubes containing fresh semisolid MSP, a portion of the last culture was diluited and seeded onto CR substrate slabs. After 1 week, the colonies with typical morphology of Azospirillum genus were isolated on Nutrient Agar or CR and were evaluated for their capability of fixing atmospheric nitrogen.

The isolated strains are maintained on Nutrient Agar containing slabs or test tubes or freezing liquid cultures in MSP admixed with 10% of dimethylsulfoxide and 20% of glycerol.

Formulations and treatment modalities

Azospirillum NCIMB 40487 and NCIMB 40488 strains are generally used in the treatment of crops and namely as cells capable of regenerating new colonies.

They can be used as such, as aqueous suspensions, or as formulations prepared using either liquid or solid inert carriers.

A very important aspect in order to obtain a high biological activity is the formulation type with which the microorganism is applied to seeds.

In the past, organic supports were used, such peat (for example, ZEANIT, HELIBIOAGRI), soil or liquid culture substrates.

However, such supports are affected by the drawback that they cannot be perfectly sterilized and consequently, during storage, some polluting microorganisms gain advantage over Azospirillum causing it to completely disappear after a short time period.

The above drawbacks were overcome by means of the development of a novel formulation comprising Azospirillum NCIMB 40487 or NCIMB 40488, or a mixture thereof and celite.

Such a formulation enables Azospirillum NCIMB 40487 and NCIMB 40488 to survive for long time at room temperature and can be used for seed dressing and for treating crop growth soils both in nursery garden or seed-bed, and in open field.

The use of celite for the purpose of Azospirillum NCIMB 40487 and NCIMB 40488 survival under storage conditions at room temperature resulted to be decidedly more advantageous as compared to other supports taken into consideration, for example, vermiculite, sepiolite or kaolin co-adjuved by adhesion promoters, such as gum arabic, carboxymethylcellulose or poly(vinyl alcohol) (PVA).

As adhesion promoter, on the contrary, poly(vinyl alcohol), in particular, PVA N300, proved to be the best one, because, besides securing a good survival of beneficial microorganisms, it does not favour the development of most phytopathogenous fungi present in agricultural land.

The seeds are first treated with the adhesion promoter and subsequently with the carrier onto which the aqueous suspension of Azospirillum was adsorbed.

The application doses are not critical, in fact it is only necessary that the bacteria are present in a large enough amount in order to come into contact with the roots of the plants and then colonize them.

In general, the following doses may be used:

-- in case of seed dressing, from $10^3$ to $10^7$ cells for each seed;

-- in the case of the treatment of the soil of seed-beds, from $10^4$ to $10^9$ cells per each soil litre;

-- in the case of the open field treatment, from $10^7$ to $10^{15}$ cells per hectare;

The bacterial Azospirillum NCIMB 40487 and NCIMB 40488 strains can be used either as individual strains, or as mixtures with each other.

Biological activity

The observations carried out during the growth of plants originated from seeds treated with cells of Azospirillum NCIMB 40487 or NCIMB 40488, or grown in soil to which said microorganism were added, made it possible the following positive effect to be detected:

-- generalized increase in plant size;

-- better developed roots.

Such effects are shown in particular by plants grown under strong water stress conditions.

The interaction between Azospirillum NCIMB 40487 and NCIMB 40488 and the plant with the result that the vegetation conditions of the latter are modified, occurs at root level where said microorganism preferentially grow. In fact, we were able to verify that such microorganisms colonized and hence plentifully reproduce themselves on the roots of the plants originated from seeds or grown on soils to which Azospirillum NCIMB 40487 and NCIMB 40488 were added.

For exemplifying purposes, the values are reported of colonization by Azospirillum NCIMB 40487 on the roots of 8 different corn cultivars grown on sterile sand and originated from commercial seeds treated with chemical fungicidal and insecticidal agents (Captan and Methoxychlor), as well as from the same seeds after removal of the chemical treatment agent with acetone and subsequent admixing with the microorganism formulated with celite.

The colonization values, expressed as number of colonies forming units (CFU) per each root after a 9-day growth of the plants in sterile sand at 21°C are illustrated in Table 5:

Table 5

| Corn cultivar | As available on the market | Washed |
|---|---|---|
| Markober | $6.08 \times 10^6$ | $1.75 \times 10^6$ |
| Mercury | $7.25 \times 10^5$ | $8.30 \times 10^6$ |
| Mark | $9.58 \times 10^5$ | $1.08 \times 10^7$ |
| Zelig | $1.77 \times 10^6$ | $8.30 \times 10^4$ |
| Harvest | $1.78 \times 10^7$ | $4.29 \times 10^5$ |
| Sirio | $6.50 \times 10^6$ | $3.11 \times 10^6$ |
| Laser | $8.16 \times 10^6$ | $2.13 \times 10^6$ |
| Amstrong | $4.25 \times 10^6$ | $6.43 \times 10^6$ |

In most cases, the presence of the chemical treatment does not influence to a considerable extent the colonization capability by the microorganism used. Also the corn variety used results to unimportant.

Another important requisite for the bacteria to be capable of performing their function is their capability of surviving and reproducing themselves under open field conditions.

In fact, the variability of the results reported in scientific literature can be attributed to the poor capability of used strains to survive in the presence of competitors present in the agricultural field used.

NCIMB 40487 and NCIMB 40488 strains formulated on celite, confirmed to have an extremely good capability of surviving, as it was demonstrated by the results of the following experimental tests.

The tests were carried out on corn grown in pots (18 cm x 16 cm) containing 3.3 kg of clayey-slimy agricultural soil with 0.9% of organic substance and a ratio of C:N of 8:1. The soil was supplemented with 0-100-200 mg of nitrogen per kg. The seeds were sown to a depth of 2.5 cm. Each pot received 7 seeds. 15 days later, the number of plants was thinned out to 4 seedlings per pot. The plants were grown outdoor. Before sowing the seeds were dressed with a formulate of Azospirillum on celite made resistant to kanamycin by labelling with transposon Tn 5. Each seed of corn cv. Derek (Dekalb) received $10^6$ bacteria.

The count of the bacteria present on the roots was carried out as follows: 10-15 cm long samples were collected and, after being washed under running water in order to remove the coarsest soil particles, they were suspended in 20 ml of 0.01 M phosphate buffer pH 7 and the suspensions were kept with stirring for 1 hour. The suspension was serially diluted and distributed on agarised growth substrate (MSP or Nutrient Agar), to which cycloheximide (100 micrograms/ml) and kanamycin (50 micrograms/ml) were added. After a 7-day incubation, colonies were counted. As other bacteria grew on the substrates used, the identification of Azospirillum was possible thanks to the typical morphology of the colonies.

The experiment was carried out by following the randomized block scheme with 4 replicates per thesis. Groups of 4 pots were sampled at each test time (15 and 30 days of sowing). The technique used made it possible a minimal number of 103 bacteria per each seed or root sample to be detected.

Table 6 relates to the plants grown in fertilizer-free soil and reports the results from the count of colonies of Azospirillum NCIMB 40487 and NCIMB 40488, as compared to the strain isolated from the commercial formulate ZEANIT (ZN).

Table 6

| Microorganism | 0 days | 15 days | 30 days |
|---|---|---|---|
| NCIMB 40487 | $1.4 \times 10^6$ | $1.2 \times 10^5$ | $2.1 \times 10^6$ |
| NCIMB 40488 | $1.4 \times 10^6$ | $1.4 \times 10^4$ | $<10^3$ |
| ZN | $1.4 \times 10^6$ | $<10^3$ | $<10^3$ |

Table 7 relates to plants grown in soil with 100 mg/kg of nitrogenous fertilizer and reports the results from the count of colonies of Azospirillum NCIMB 40487 and NCIMB 40488, as compared to the strain isolated from the commercial formulate ZEANIT (ZN).

Table 7

| Microorganism | 0 days | 15 days | 30 days |
|---|---|---|---|
| NCIMB 40487 | $1.4 \times 10^6$ | $3.4 \times 10^5$ | $1.8 \times 10^6$ |
| NCIMB 40488 | $1.4 \times 10^6$ | $1.4 \times 10^4$ | $<10^3$ |
| ZN | $1.4 \times 10^6$ | $<10^3$ | $<10^3$ |

Table 8 relates to plants grown in soil with 200 mg/kg of nitrogenous fertilizer and reports the results from the count of colonies of Azospirillum NCIMB 40487 and NCIMB 40488, as compared to the strain isolated from the commercial formulate ZEANIT (ZN).

Table 8

| Microorganism | 0 days | 15 days | 30 days |
|---|---|---|---|
| NCIMB 40487 | $1.4 \times 10^6$ | $1.2 \times 10^6$ | $1.4 \times 10^6$ |
| NCIMB 40488 | $1.4 \times 10^6$ | $1.5 \times 10^4$ | $<10^3$ |
| ZN | $1.4 \times 10^6$ | $<10^3$ | $<10^3$ |

In order to explain the positive effects of Azospirillum on plants, different action mechanisms were suggested:

(1) fixation of atmospheric nitrogen;

(2) stimulation of root system, caused by the synthesis of vegetable hormones by the microorganism: this increase in root system leads to an increased capacity of absorption of mineral substances and to an increased resistance to water lack;

(3) simultaneous modification of soil microflora, and

(4) production of siderophores.

Besides displaying the capability of increasing the weight of corn seedlings grown in agricultural Soil, Azospirillum NCIMB 40487 and NCIMB 40488 according to the present invention, are capable of producing the vegetable hormone 3-indoleacetic acid (IAA), of growing and persisting on the rizosphere of corn mais, performing the nitrogenasic function and of inducing the development of root system.

Azospirillum NCIMB 40487 and NCIMB 40488 strains are natural products which, when used as phytostimulants, result to be harmless for warm-blooded animals and environmentally safe, i.e., they do not alter or pollute environment, either as such, or through their degradation products.

Some examples are given now for illustrative purposes, which in no way should be understood as being limitative of the present invention.

Example 1

Preparation of formulates

Azospirillum NCIMB 40487 and NCIMB 40488I are grown in agitated cultures (250 revolutions per minute) of liquid substrate MSP at 32°C, up to the stage of steady-state growth (48 hours).

After cell centrifuging and washing thereof with 0.01 M phosphate buffer, the bacteria are suspended in MSP at the end concentration of $10^8$ cfu/ml and are used in order to impregnate sterile celite. The impregnation operation is carried out by adding, under sterile conditions, 1 litre of bacterial suspension to each kg of celite.

The formulate on celite was applied to the seed previously moistened with a solution of PVA N300 at 2%.

Example 2

An investigation was carried out into some inorganic and organic carriers in powder form in order to verify their usefulness and effectiveness in keeping Azospirillum alive in suitable formulates for bacterial seed dressing.

The following were taken into consideration:

-- Kaolin Argirec B22;
-- Corn starch Globe 03401;
-- Sepiolite 200 ASTM;
-- Celite 209;
-- Finely ground vermiculite.

The above substrate were evaluated for their intrinsic capability to adsorb and keep alive the Azospirillum strains under investigation; liquid cultures of AZD 94 and AZD 167 were adsorbed onto such carriers and after a storage period at 5°C and 25°C, the vitality and purity of the formulate were evaluated.

The results demonstrate that, while the several inorganic substrates do not display any special problems, the corn starch-based formulate is unsuitable, because it is too easily subject to pollution by foreign microorganisms.

As regards the applicability to the seed, it was observed that wheat, barley and corn seeds pre-treated with a 2% solution of poly(vinyl alcohol) N300 are easily dressed with the formulates in powder form, retaining good free-flowing properties.

The capability of colonization and survival of microorganisms on the root system of plants originated from seeds treated with the formulates supported on the several carriers, resulted to be better when celite-based formulates were used. Such a formulate is in fact capable of dressing the seed with bacteria, perfectly adhering onto its surface, and of inducing a colonization of $10^5$ bacteria/g of roots, a level which is considered adequate in order to effectively operate on the plant.

Therefore, the celite-based formulate was chosen in order to be submitted to a deeper study of long-term stability (up to 3 months) under different storage conditions (room temperature and 5°C).

The analysis of the formulate was carried out by indirect way, by treating corn seeds and evaluating the level of colonization of root system of developed seedlings.

The results of the evaluations carried out are summarized in following Table 9.

11

## Table 9

| | CFU/seed | $t_0$ | $t_1$ | | $t_2$ | | $t_3$ | $t_4$ | $t_5$ |
|---|---|---|---|---|---|---|---|---|---|
| | | | 5° C | 25° C | 5° C | 25° C | 25° C | 25° C | 25° C |
| NCIMB 40487 | $3.7 \times 10^6$ | $4.85 \times 10^6$ | $<10^2$ | $2.74 \times 10^6$ | $5 \times 10^2$ | $5.78 \times 10^4$ | $3.37 \times 10^7$ | $1.49 \times 10^6$ | $1.04 \times 10^7$ |
| NCIMB 40488 | $3.3 \times 10^6$ | $1.86 \times 10^6$ | $2.6 \times 10^5$ | $1.44 \times 10^6$ | $4.5 \times 10^4$ | $1.25 \times 10^5$ | $1.4 \times 10^6$ | $1.29 \times 10^5$ | $8.33 \times 10^4$ |

The results are expressed as cfu/gram of root

$t_0$     Titre at formulation time
$t_1$     Titre 2 weeks after formulation
$t_2$     Titre 4 weeks after formulation
$t_3$     Titre 6 weeks after formulation
$t_4$     Titre 10 weeks after formulation
$t_5$     Titre 14 weeks after formulation

EP 0 570 079 A1

After a 14-week storage at room temperature, the formulate resulted to be capable of inducing a colonization of $10^4$-$10^7$ bacteria/root g, i.e. , the same value as induced by the formulate soon after being prepared. The storage at 50°C is Less suitable, because already after 2 weeks, the formulate induces a colonization of only $10^2$ /g of roots.

Example 3

Production of 3-indoleacetic acid (IAA)

The capability of NCIMB 40487 and NCIMB 40488 strains to produce the vegetable hormone IAA was evaluated by the HPLC technique.

The cultures of Azospirillum were prepared in liquid MSP substrate, to which 50 micrograms/mL of tryptophan was added. The analysis was carried out on the acidified extracts (pH 2.8 with 1 N hydrochloric) of the supernatants of cultures grown for 48 hours in the dark. After extraction with 4-methyl-2-pentanone solvent and extract concentration to dryness, the residue was collected with methanol and analyzed with an RP18 column. The results are reported in following Table 10.

Table 10

| Microorganism | IAA |
|---|---|
| Azospirillum brasilense ATCC 29145 | 9.2 micrograms/ml |
| Azospirillum brasilense ATCC 29710 | 1.3 micrograms/ml |
| NCIMB 40487 | 15.3 micrograms/ml |
| NCIMB 40488 | 22.7 micrograms/ml |

These results are the average values obtained from 3 different analyses.

Example 4

Affinity with rizosphere

The affinity with rizosphere by Azospirillum NCIMB 40487 and NCIMB 40488 was evaluated as the capability to grow and persist on the rizosphere of corn plants and to carry out the nitrogenasic function.

Seeds of corn cv. Plenus (Dekalb) sterilized with 20% hypochlorite were seeded in sterile vermiculite (400 ml) moistened with 250 ml of nutrient solution free from nitrogenous salts. After a 7-day growth in a controlled-atmosphere cell at 23°C with 16 hours of light per day, onto the surface of vermiculite 10 ml was inoculated of a suspension of NCIMB 40487, NCIMB 40488 or Sp7 (ATCC 29145). 14 days later, the roots of the plants which had developed were collected, were finely ground and homogenized with phosphate buffer and an aliquot of homogenate (0.2 ml) was transferred into test tubes containing semisolid MSP. 24 hours later, the acetylene reduction assay (ARA), known to those skilled in the art in order to evaluate the nitrogen fixing capability of bacteria, was carried out. The concentration of ethylene produced was compared to that developed by ATCC 29145 strain, taken as the reference (100).

The results are the average values from 5 replicates and are reported in following Table 11.

Table 11

| Microorganism | % |
|---|---|
| ATCC 29145 | 100 |

Table 11 (continuation)

Microorganism | | %
| NCIMB 40487 | 120 |
| NCIMB 40488 | 169 |

## Example 5

A set of tests was carried out in order to determine the biostimulating activity of Azospirillum NCIMB 40487 and NCIMB 40488I on the growth of corn seedlings and of their roots, cultivated under different conditions.

Corn seeds treated with celite-based formulates of the microorganisms under study are used in order to seed 500 ml of sand, contained in a beaker of 600 ml. The sand was previously washed, dried, treated in autoclave and then was moistened with 150 ml of water per each kg of sand.

After a 30-day growth in controlled-atmosphere cell at 23°C, the root surface area of the roots was determined (the weight difference was not detectable) according to the following method (Carley, H.E. et al., Soil Science 102 289-291 1966): the roots to be examined are washed under running water and are allowed to dry over night at room temperature, laid on a flat surface. They are then dipped for 15 seconds in 3 N HCl, are kept overhanging for 10 minutes in order to eliminate the excess of hydrochloric acid, and are transferred into distilled water for 3 hours.

The wash water is titrated with 0.1 N NaOH.

The sodium hydroxide amount used in order to neutralize the hydrochloric acid adsorbed on root surface is proportional to the same surface area.

The results are reported in Table 12.

Table 12

| | Inoculum (cfu/seed) | Surface area (ml of NaOH) | Increase (%) |
|---|---|---|---|
| Comparison Test | 10.93 | | 100 |
| NCIMB 40487 | $2 \times 10^8$ | 11.62 | 106 |
| | $2 \times 10^7$ | 15.48 | 142 |
| | $2 \times 10^6$ | 14.22 | 130 |
| | $2 \times 10^5$ | 12.30 | 112 |
| | $2 \times 10^4$ | 11.96 | 109 |
| Comparison Test | 9.17 | | 100 |
| NCIMB 40488 | $1.2 \times 10^8$ | 9.75 | 106 |
| | $1.2 \times 10^7$ | 10.11 | 110 |
| | $1.2 \times 10^6$ | 8.02 | 87 |
| | $1.2 \times 10^5$ | 8.55 | 93 |
| | $1.2 \times 10^4$ | 6.70 | 73 |

## Example 6

Corn seeds dressed with celite-based formulates of NCIMB 40487 and NCIMB 40488 strains are used in order to sow 500 ml of agricultural soil (84% sand, 11% slime, 5% clay, 3.8% organic matter, 0.17% total nitrogen, pH 5.7) contained inside pots.

After a 21-day growth in greenhouse at 230°C, the root surface area of the roots was determined (the weight difference was not appreciable), by following the Carley's method. The results are reported in Table 14.

Table 14

|  | Inoculum (cfu/seed) | Surface area (ml of NaOH) | Increase (%) |
|---|---|---|---|
| Comparison Test | 9.31 |  | 100 |
| NCIMB 40487 | $5.6 \times 10^8$ | 10.09 | 108 |
|  | $5.6 \times 10^7$ | 9.73 | 104 |
|  | $5.6 \times 10^6$ | 9.98 | 107 |
|  | $5.6 \times 10^5$ | 10.43 | 112 |
| Comparison Test | 9.91 |  | 100 |
| NCIMB 40488 | $3.1 \times 10^8$ | 10.90 | 109 |
|  | $3.1 \times 10^7$ | 8.88 | 90 |
|  | $3.1 \times 10^6$ | 10.37 | 105 |
|  | $3.1 \times 10^5$ | 11.47 | 116 |

Example 7

For the tests in hydroponic crop, the plants were cultivated by placing the pre-germinated corn seeds on metal nets fastened onto the surface of 400 ml of an Hoagland solution not containing combined nitrogen and inoculated with Azospirillum NCIMB 40487 and NCIMB 40488 at the end concentration of $10^6$ and $10^7$ cells/ml.

After a 14-day growth in controlled-atmosphere cell at 23°C, the roots were weighed. The results are reported in Table 15.

Table 15

| Treatment | Dose (cfu/mL) | Fresh weight (g) | Increase (%) |
|---|---|---|---|
| Comparison Test | 2.64 |  | 100 |
| NCIMB 40487 | $10^7$ | 5.87 | 222 |
|  | $10^6$ | 4.50 | 170 |

Table 15 (continuation)

| Treatment | Dose (cfu/mL) | Fresh weight (g) | Increase (%) |
|---|---|---|---|
| NCIMB 40488 | $10^7$ | 6.22 | 236 |
|  | $10^6$ | 4.35 | 165 |

Example 8

In order to evaluate the effect of water lack, the seeds treated with celite-based formulates of Azospirillum NCIMB 40487 and NCIMB 40488 were sown in soil (69% sand, 12% slime, 19% clay, 0.9% organic matter, 0.07% total nitrogen) containing a moisture amount equivalent to 70% of its water capacity. After 10 days of growth, the amount of restored water amount was reduced until 30% of water capacity of soil was reached. After 15 days under these conditions, the seedlings were cut at their base and their dry weight was measured. The results are reported in following Table 16.

Table 16

| Treatment | Dry weight (mg) | Increase (%) |
|---|---|---|
| Comparison Test | 268 | 100 |
| NCIMB 40487 | 342 | 128 |
| NCIMB 40488 | 359 | 134 |

**Claims**

1.  Method for stimulating the growth of corn plants, which method consists in treating the seeds, the roots or the soil in which they grow, with a suitable amount of a culture of Azospirillum NCIMB 40487 and NCIMB 40488, or of a mixture of both of them, as such, or as a suitable composition comprising either solid or liquid inert carriers or other additives.

2.  Biostimulating composition for agricultural crops comprising Azospirillum NCIMB 40487 and NCIMB 40488, or a mixture of both of them and celite.

3.  Azospirillum NCIMB 40487 and NCIMB 40488 and biologically pure cultures thereof.

4.  Method for dressing seeds with Azospirillum NCIMB 40487 or NCIMB 40488 or a mixture of both of them, which comprises a preliminary treatment of the seeds with poly(vinyl alcohol) PVA N300 and a subsequent treatment with the composition according to claim 2.

| | | |
|---|---|---|
| European Patent Office | **EUROPEAN SEARCH REPORT** | Application Number |
| | | EP 93 20 1381 Page 1 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | CHEMICAL ABSTRACTS, vol. 110, no. 17, 24 April 1989, Columbus, Ohio, US; abstract no. 151359q, E.FALLIK ET. AL. 'Identification and quantification of IAA and IBA in Azospirillum brasilense-inoculated maise roots' page 439 ; & SOIL BIOL. BIOCHEM. vol. 21, no. 1, 1989, pages 147 - 153 --- | | A01N63/00 C12N1/20 A01C1/06 //(A01N63/00, 25:08) (C12N1/20, C12R1:01) |
| A | CHEMICAL ABSTRACTS, vol. 95, no. 3, 20 July 1981, Columbus, Ohio, US; abstract no. 23473g, Y.KAPULNIK ET. AL. 'Yield increases in summer cereal crops in Israeli fields inoculated with Azospirillum' page 562 ; * abstract * & EXP. AGRIC. vol. 17, no. 2, 1981, pages 179 - 187 --- | | |
| A | CHEMICAL ABSTRACTS, vol. 110, no. 13, 27 March 1989, Columbus, Ohio, US; abstract no. 113624q, F.R.WAREMBOURG ET. AL. 'Plant-microorganism interactions and the rhizosphere energy status. Role of microflora' page 592 ; & REV. ECOL. BIOL. SOL vol. 24, no. 3, 1987, pages 473 - 483 --- -/-- | | TECHNICAL FIELDS SEARCHED (Int. Cl.5 ) A01N A01C |

The present search report has been drawn up for all claims

| Place of search | Date of completion of the search | Examiner |
|---|---|---|
| THE HAGUE | 27 AUGUST 1993 | DONOVAN T.M. |

EPO FORM 1503 03.82 (P0401)

European Patent Office

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 91, no. 5, 30 July 1979, Columbus, Ohio, US; abstract no. 35846m, T.M. TIEN ET. AL. 'Plant growth substances produced by Azospirillum brasilense and their effect on the growth of pearl millet (Pennisetum americanum L.)' page 328 ; & APPLI. ENVIRON.MICROBIOL. vol. 37, no. 5, 1979, pages 1016 - 1024 --- | | |
| A | TRENDS IN BIOTECHNOLOGY vol. 7, no. 2, February 1989, pages 39 - 44 J.W.KLOEPPER 'Free-living bacterial inocula for enhancing crop productivity' --- | | |
| A | APPLIED MICROBIOLOGY AND BIOTECHNOLOGY vol. 32, no. 4, January 1990, pages 473 - 478 J.FAGES 'An optimised process for manufacturing an Azospirillum inoculant for crops' | | TECHNICAL FIELDS SEARCHED (Int. Cl.5) |

-----

The present search report has been drawn up for all claims

| Place of search | Date of completion of the search | Examiner |
|---|---|---|
| THE HAGUE | 27 AUGUST 1993 | DONOVAN T.M. |

EPO FORM 1503 03.82 (P0401)